# EUROPEAN PATENT SPECIFICATION

(11) **EP 1 602 925 B1**
(45) Date of publication and mention of the grant of the patent: **30.04.2008**
(21) Application number: 05010493.4
(22) Date of filing: 13.05.2005
(51) Int. Cl.: A61L 24/10, A61L 24/04, A61F 2/06, A61L 31/04

(54) **System for treatment of aneurysmal tissue**
System zur Behandlung von Aneurysmen
Système pour le traitement d'aneurysmes

(30) Priority: 27.05.2004 US 575008 P; 19.07.2004 US 894945
(43) Date of publication of application: 07.12.2005
(73) Proprietor: Medtronic Vascular, Inc., Santa Rosa, California 95403 (US)
(72) Inventor: Letort, Michel, 01280 Prevessins (FR)
(74) Representative: Zimmermann & Partner

(56) References cited:
- WO-A-00/67774
- WO-A-01/66017
- WO-A-99/66964
- US-A- 5 583 114
- BARROWS T H ET AL: "Evaluation of a new tissue sealant material: Serum albumin crosslinked in vivo with polyethylene glycol" TRANS ANNU MEET SOC BIOMATER INT BIOMATER SYMP; TRANSACTIONS OF THE ANNUAL MEETING OF THE SOCIETY FOR BIOMATERIALS IN CONJUNCTION WITH THE INTERNATIONAL BIOMATERIALS SYMPOSIUM 1996 SOC FOR BIOMATERIALS, ST. LOUIS PARK, MN, USA, vol. 1, 1996, page 8, XP009055814
- TRUONG M T ET AL: "In vitro analysis of mechanical properties of a new tissue sealant material: Polyethylene glycol crosslinked serum albumin" TRANS ANNU MEET SOC BIOMATER INT BIOMATER SYMP; TRANSACTIONS OF THE ANNUAL MEETING OF THE SOCIETY FOR BIOMATERIALS IN CONJUNCTION WITH THE INTERNATIONAL BIOMATERIALS SYMPOSIUM 1996 SOC FOR BIOMATERIALS, ST. LOUIS PARK, MN, USA, vol. 2, 1996, page 73, XP009055815

## Description

### FIELD OF THE INVENTION

The field of the invention is the treatment of vascular abnormalities.

### BACKGROUND OF THE INVENTION

Aortic aneurysms pose a significant medical problem for the general population. Aneurysms within the aorta presently affect between two and seven percent of the general population and the rate of incidence appears to be increasing. This form of atherosclerotic vascular disease (hardening of the arteries) is characterized by degeneration in the arterial wall in which the wall weakens and balloons outward by thinning. Generally, until the affected artery is removed or bypassed, a patient with an aortic aneurysm must live with the threat of aortic aneurysm rupture and death.

One clinical approach for patients with an aortic aneurysm is aneurysmal repair by endovascular grafting. Endovascular grafting involves the transluminal placement of a prosthetic arterial stent graft in an endoluminal position. To prevent rupture of the aneurysm, a stent graft of tubular construction is introduced into the aneurysmal blood vessel, typically from a remote location through a catheter introduced into a major blood vessel in the leg.

When inserted and deployed in a vessel, a stent graft acts as a prosthesis to maintain and restrict blood flow through the vessel. The stent graft typically has the form of an open-ended tubular element and most frequently is configured to enable its expansion from an outside diameter which is sufficiently small to allow the stent graft to traverse the vessel to reach a site where it is to be deployed, to an outside diameter sufficiently large to engage the inner lining of the vessel for retention at the site.

Despite the effectiveness of endovascular grafting, once the aneurysmal site is bypassed, the aneurysm remains. The aortic tissue can continue to degenerate such that the aneurysm still increases in size due to ongoing thinning of the medial connective tissue architecture of the aorta and degradation of elastin. Thus there is a desire in the art to achieve a greater success of aneurysmal repair and healing. It is therefore an object of the present invention to overcome the problems associated with the prior art at least partially.

WO 01/66017 discloses the use of cross-linked blood proteins for the treatment of aneurysms. There is however no disclosure of the combined use of a stent-graft.

### SUMMARY OF THE INVENTION

This object is solved by an aneurismal treatment system according to claim 1. Further aspects, features, advantages and details of the present invention are apparent from the dependent claims, the description and the accompanying drawings. Embodiments according to the present invention address the problem of aneurysm support and repair, particularly the problem of continued breakdown of vessel tissue. A consequence of such continued breakdown is rupture of the aneurysm. Embodiments according to the present invention provide a method for supporting or bolstering the aneurismal site in an individual with cross-linked proteins from the individual's blood.

Thus, there is provided a method for treating an aneurysmal site in a blood vessel, comprising: withdrawing blood from an individual; isolating proteins from the blood; cross-linking the proteins; and delivering the cross-linked proteins to the aneurysmal site by a delivery means, e.g., such as a catheter. In addition, certain embodiments of the invention may further comprise deploying a stent at the aneurysmal site; where the cross-linked proteins are delivered to the space between an exterior wall of the stent and an interior wall of the aneurysmal site.

### BRIEF DESCRIPTION OF THE DRAWINGS

Figure 1 is a schematic view of a human aorta with an where a stent graft has been deployed therein.

Figure 2 is a flow chart showing generally the steps according to one embodiment of the present invention.

Figure 3 is a partial sectional view of a descending aorta with a stent, a delivery catheter and cross-linked proteins placed within the aneurysmal sac.

### DETAILED DESCRIPTION

Stabilizing and treating an aneurysmal site includes bolstering and stabilizing the aneurismal area of an individual by using crosslinked proteins from the individual's blood. A stent graft is first implanted in the individual isolating the region between the outer wall of the stent graft and the inner wall of the aneurismal region of the blood vessel. The cross-linked proteins are then delivered to this isolated region.

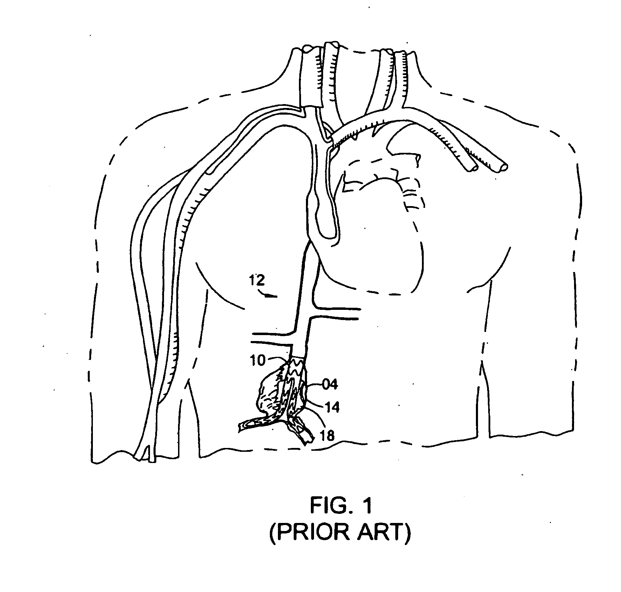

Referring initially to Figure 1, there is shown generally an aneurysmal blood vessel; in particular, there is an aneurysm of the aorta 12, such that the blood vessel wall 04 is enlarged. This is an aneurysmal site 14. The aneurysmal site 14 includes an aneurysmal bulge or sac 18. If left untreated, the aneurysmal sac 18 may continue to deteriorate, weaken, increase in size, and eventually tear or burst.

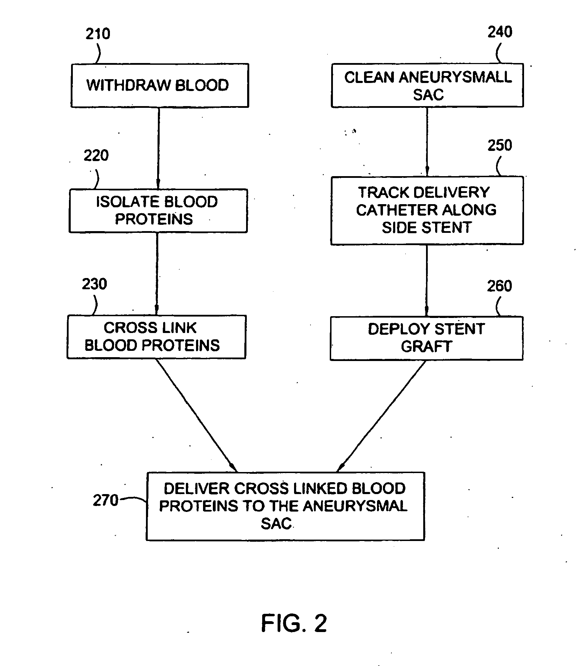

Figure 2 is a flow chart showing generally the steps of a process 200 according to one embodiment of the present invention. First, in step 210, blood is withdrawn from an individual in a typical manner such as venipuncture using, e.g., a needle, syringe, and one or more evacuated tubes. Such venipuncture induces little to no discomfort, and can be performed on an outpatient basis or shortly before or concurrently with the cleaning (240), tracking (250) and/or deploying (260) steps.

In step 220, plasma from the blood is isolated, and the plasma proteins are then isolated from the plasma. Isolating plasma from whole blood is most easily accomplished by centrifuging the withdrawn whole blood, for example, by using a blood platelet processor such as a Medtronics Magellan® Autologous Platelet Separator system. The red cells in the whole blood sediment at the bottom of the centrifuge tube, the white cells of the blood sediment at the top of the red blood cell layer forming a "buffy coat", and the plasma forms the top layer. Blood comprises roughly 50% plasma in an average adult. Blood plasma is approximately 92% water, 6-8 % protein, 0.8% salt, 0.6% lipid and 0.1% glucose.

Blood plasma serves several important functions, including transporting materials needed by the cells of the body as well as transporting waste products and other materials that must be removed from the body, including various ions (Na⁺¹, Ca⁺², HCO₃⁻), amino acids, glucose, organic acids, cholesterol, hormones, urea and other wastes. Most of these materials are in transit from a place where they are added to the blood such as the intestine or the liver to places where they will be removed from the blood, such as by the cells of the body, the kidney and the skin.

Proteins make up 6-8% of the blood, and are about equally divided between serum albumin and a great variety of other proteins (over 450) such as the serum globulins (such as alpha-, beta-, and gamma-globulins). Serum albumin is made in the liver, binds small molecules for transport through the blood, and helps maintain the osmotic pressure of the blood. The alpha and beta serum globulins transport thyroxine, retinal and transferrin. Most antibodies are gamma globulins. A process according to the present invention provides cross-linking of plasma proteins in general, but particularly provides cross-linking of serum albumins.

In the next step, step 230, the proteins from the blood, primarily serum albumins, are cross-linked. Most cross-linking reagents have two reactive groups connected by a flexible "spacer" arm. Some cross-linkers, homo-bifunctional cross-linkers, have the same reactive groups at both ends. Others, hetero-bifunctional cross-linkers, have different reactive groups at the ends. Yet another type of cross-linker contains additional functional groups permitting these cross-linkers to serve another purpose, for example, carry a detectable label. There also are several trifunctional cross-linkers that allow trimeric complexes to be made.

Most protein cross-linking reactions are side-chain reactions and are nucleophilic, resulting in a portion of the end of the cross-linker being displaced in the reaction (the leaving group). Nucleophilic attack is dependent on pH, the temperature and ionic strength of the cross-linking buffer. When the buffer is one to two pH units below the pKa of the side chain, the species is highly protonated and is most reactive; one to two pH units above the pKa, the species is not protonated and not reactive. The majority of proteins have lysines available at the surface of the protein, providing primary amines. Some proteins have cysteines that are not involved in disulfide bonds, providing free sulfhydryls. These are the two most commonly used groups in protein cross-linking strategies. Cross-linking strategies may also use carbohydrates, carboxyls or other reactive functional groups. The cross-linking groups of the present invention are, of course, biocompatible.

Factors to be considered for protein cross-linking are pH, salt concentration, additives, temperature, protein concentration, the number of reactive functional groups on the surface of the proteins, cross-linker spacer arm length, and conjugation buffer composition.

The most common class of cross-linkers contains N-hydroxysuccinimide esters (NHS esters) that react with primary amines (lysine and amino termini). Because lysine residues are abundant on the surface of most proteins, these cross-linkers will bind efficiently to almost any protein, including albumin. NHS ester reactions are carried out at about pH 7-9. Reactivity of the lysine group increases as the pH increases to 9, but competing NHS hydrolysis also is favored with pH increase. Cross-linkers that contain NHS esters are not water-soluble; however, the sulfonated forms of NHS esters (e.g., sulfo-NHS esters) are water soluble and are more stable than non-sulfonated NHS esters.

Maleimides, haloacetyls and pyridyl disulfides are common thio-reactive groups. Maleimide-containing bifunctional cross-linking reagents are most popular for specific controlled and stable conjugation methods. Maleimides react preferentially with sulfhydryls at about pH 6.5-7.5. At higher pH, maleimides will cross-react with amines. Maleimide compounds are not stable in solution for extended periods. They must be processed, frozen or lyophilized as soon as possible. Haloacetyl cross-linkers are stable in solution, but they are somewhat less specific to the sulfhydryl reaction. Pyridyl disulfides are reactive at pH 8.0 or higher and produce a mixed disulfide that can undergo further reduction to break the cross-link.

Often it is desirable to modify a protein to generate free sulfhydryl groups. The sulfhydryls then act as a convenient "handle" for generating specific cross-links to the protein. Popular sulfhydryl modifiers include N-succinimidyl-S-acetylthioacetone (SATA), N- succinimidyl-3-[2-pyridyldithio]-propionamido (SPDP), and 2-iminothiolane (Traut's Reagent). These compounds modify primary amines to create free or protected sulfhydryls in place of the amines. Various reducing agents are available for generating free sulfhydryls from disulfides within the protein. They vary in reducing strength (tris(2-carboxyethyl)phosphine HCl (TCEP)>dithiothreitol (DTT)>mercaptoethanol (ME)>cysteine) and the ability to cleave in hydrophilic (TCEP) and hydrophobic (DTT) protein regions. Free sulfhydryls are favored at a pH less than 7.0, where disulfide formation is favored above pH 7.5. Addition of ethylene diamine tetraacetic acid (EDTA) can prevent oxidation of sulfhydryls by trace metals, and degassing buffers further protects sulfhydryls from oxidation. Sulfhydryls are not stable in solution for extended periods and should be processed as soon as possible. Desalting is the best approach for removing sulfhydryl derivation reagents and reductants because it can be accomplished quickly.

Carbohydrates often are oxidized with sodium periodate to form aldehyde groups. The aldehyde groups can be reacted with hydrazines to form a stable cross-link. Amines are also reactive with oxidized carbohydrate, but they typically require the addition of a reductant to form a stable cross-link.

Using 1-ethyl-3(3-dimethylaminopropyl)-carbodiimide (EDC). carboxylate groups can be coupled to primary amines at about pH 4-7. EDC is a zero-length cross-linker that reacts with carboxyls and activates them to couple to primary amines, forming amide bonds.

In step 240, the aneurismal sac is cleared of debris. Typically, the aneurismal sac contains old thromboses, fibrous tissue and other cellular debris that, preferably, are removed before the delivery of the cross-linked proteins. Cleaning of the aneurismal sac can be performed by different methods, in particular by using a biocompatible enzymatic solution comprising thrombolytic enzymes (streptokinase, urokinase). After gentle injection into the sac--the blood flow having been interrupted or bypassed using catheters and/or balloons--the thrombus is then liquefied after a given period of time. The liquefaction process also can be extended to mechanically increase the contact with the enzyme and reduce into smaller parts the particles that are not subject to enzyme degradation. After liquefying the contents of the sac, the liquid and debris, are aspirated through a conventional larger diameter catheter. The area can then be rinsed and the rinsing solution aspirated as well.

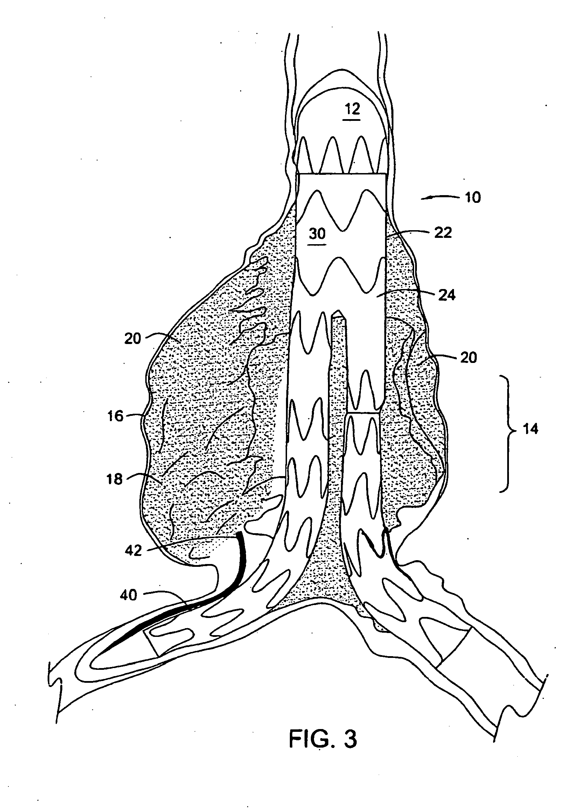

Referring still to Figure 2, once blood proteins have been isolated and cross-linked, they can be delivered to the aneurysmal site. To do so, first a delivery means, such as a catheter, is tracked through the vascular system of an individual by methods known in the art, so that the distal portion of the catheter resides in the aneurysmal portion of the aorta (250). In some embodiments, the catheter may be a double- or triple-lumen catheter, where one lumen may be used to contain a fiber optic means to view the aneurysmal sac. Once the distal end of the delivery means is residing the aneurysmal sac portion of the aorta, a stent graft is deployed spanning the aneurysm (260) (see also Figure 3).

In step 260, a stent graft is deployed at the aneurismal site, isolating the region outside of the stent graft, immediately adjacent the blood vessel wall in the aneurismal sac. Stent grafts are designed to be deployed and expanded in different ways. A stent graft can be designed to self expand upon release from its delivery system, or it may require application of a radial force through the delivery system to expand the stent to the desired diameter. Self-expanding stent grafts are compressed prior to insertion into the delivery device and released by the practitioner when correctly positioned within the stricture site. After release, the stent graft self-expands to a predetermined diameter and is held in place by the expansion force of the device against the interior wall of the vessel. Stent grafts that require mechanical expansion by the surgeon are commonly deployed by a balloon-type catheter. Once positioned within the stricture, the stent graft is expanded *in situ* to a size sufficient to fill the lumen and prevent restenosis. Various designs and other means of expansion also have been developed for stent graft delivery.

Stents and stent grafts are well known in the art. The stent portion of a stent graft may be biodegradable and include a therapeutic agent formulated therewith (i.e., embedded in the biodegradable polymer or covalently bound to the biodegradable polymer); alternatively, the stent can be either biodegradable or non-biodegradable with or without a therapeutic agent formulated with a compound that is used to coat or is otherwise applied to the stent.

In selecting an appropriate therapeutic agent or agents, one objective is to protect the aneurysmal blood vessel from further destruction, aid in stabilizing the cross-linked proteins, and/or promote healing. Generally, aneurysm results from the invasion of the cell wall by elastin-degradation proteins that occur naturally in the body, but for unknown reasons begin to congregate at certain blood vessel sites, attack the blood vessel structure and cause inflammation of the vessel. Generally, a plurality of enzymes, proteins and acids--all naturally occurring--interact through specific biochemical pathways to form elastin-degradation proteins or to promote the attachment or absorption of elastin- degradation proteins into the cell wall. The elastin-degradation proteins and the resulting breakdown of tissue and inflammation are leading causes of aneurysm formation.

The therapeutic agents used provide intervention in the aforementioned biochemical pathways and mechanisms, reduction in the level of the individual components responsible for aneurysmal growth, stabilization of the cross-linked proteins or elimination or limitation of the advance of the aneurysmal event. Preferably, the therapeutic agent or agents are released over time in the aneurysmal location, reducing the likelihood of further dilation and increasing the likelihood of successful repair of the aneurysm.

The maximal dosage of the therapeutic to be administered is the highest dosage that effectively stabilizes the cross-linked proteins, or inhibits elastolytic, inflammatory or other aneurysmal activity but does not cause undesirable or intolerable side effects. The dosage of the therapeutic agent or agents used will vary depending on properties of the coating, including its time-release properties, whether the coating is itself biodegradable, and other properties. Also, the dosage of the therapeutic agent or agents used will vary depending on the potency, pathways of metabolism, extent of absorption, half-life, and mechanisms of elimination of the therapeutic agent itself. In any event, the practitioner is guided by skill and knowledge in the field, and embodiments according to the present invention include without limitation dosages that are effective to achieve the described phenomena.

Once the stent graft and delivery means (for example, a catheter) are in position, the cross-linked proteins can then be delivered to the aneurysmal sac (270). Other methods known in the art also may be used to deliver the cross-linked proteins such as percutaneous laparoscopic delivery, using two or more catheters--one to deploy the stent graft and one to deliver the proteins, microinjection, or other methods known to those with skill in the art.

Moreover, the cross-linked proteins can be delivered with or without a pharmaceutically acceptable solution or diluent. For example, the cross-linked proteins may be delivered in a carrier of sterile water, normal saline or other pharmaceutically acceptable carrier, alone or in combination with a pharmaceutically acceptable auxiliary substance, such as a pH adjusting or buffering agent, tonicity adjusting agent, stabilizer, wetting agent, and the like.

Figure 3 shows the transluminal placement of a prosthetic arterial stent 30, positioned in a blood vessel 10, in this embodiment in an abdominal aorta 12. The prosthetic arterial stent spans, within the aorta 12, an aneurysmal portion 14 of the aorta 12. The aneurysmal portion 14 is formed due to a bulging of the aorta wall 16, in a location where the strength and resiliency or the aorta wall 16 is weakened. As a result, an aneurysmal sac 18 is formed of distended vessel wall tissue. The stent 30 is positioned spanning the sac 18 adjacent to inner wall 16.

The placement of the stent 30 in the aorta 12 is a technique well known to those skilled in the art, and essentially includes opening a blood vessel in the leg or other remote location and inserting the stent 30 contained inside a catheter (not shown) into the blood vessel. The catheter/stent combination is tracked through the remote vessel until the stent 30 is deployed in a position that spans the aneurysmal portion 14 of aorta 12.

Cross-linked proteins 20 are shown as having been delivered between the inner wall 16 of the aneurismal sac 18 and the outer wall 22 of the prosthetic arterial stent graft 30 through a distal end 42 of a delivery means (such as a catheter) 40.

All references cited herein are to aid in the understanding of the invention.

## Claims

1. An aneurismal treatment system comprising:
a stent graft for placement in a blood vessel adjacent an aneurismal sac; the stent graft having an interior wall and an exterior wall, the exterior wall being adjacent the inner luminal wall of the blood vessel after implantation into the blood vessel adjacent the aneurismal sac;
a biocompatible cross-linked blood protein composition, wherein the biocompatible cross-linked blood protein composition is derived in vitro from blood drawn from the patient being treated using the aneurismal treatment system; and
a delivery catheter suitable for delivering the biocompatible cross-linked blood protein composition into a space between the exterior wall of the stent graft and the aneurismal sac.

2. The system of claim 1, wherein the catheter is a double lumen catheter.

3. The system of claim 1, wherein the catheter is a triple lumen catheter.

4. The aneurismal treatment system according to any of the preceding claims wherein the biocompatible cross-linked blood protein is cross-linked using N-hydroxy esters, maleimides, haloacetyls, pyridyl disulfides, carbohydrates or 1-ethyl-3(3-dimethylaminopropyl)-carbodiimide.

5. The aneurismal treatment system according to any of the preceding claims wherein the biocompatible cross-linked blood protein composition is derived primarily from serum albumin.

6. The aneurismal treatment system according to any of claims 1 to 3 wherein the cross-linker is a N-hydroxysuccinimide ester.

7. The aneurismal treatment system according to any of the preceding claims wherein the blood protein composition is modified prior to cross-linking by generating sulfhydryl groups on the blood proteins.

8. The aneurismal treatment system according to any of the preceding claims wherein the blood protein composition is modified prior to cross-linking by generating primary amine groups on the blood proteins.

9. The aneurismal treatment system according to anyone of the preceding claims wherein the aneurysm being treated using the aneurismal treatment system is an aortic aneurysm.

## Patentansprüche

1. Aneurysmabehandlungssystem, umfassend:
ein Stenttransplantat zur Anordnung in einem Blutgefäß, welches sich benachbart zu einem Aneurysmasack befindet; wobei das Stenttransplantat eine innere Wand und eine äußere Wand aufweist, wobei sich die äußere Wand nach Implantation in das Blutgefäß, welches sich benachbart zu dem Aneurismasack befindet, benachbart zu der inneren Lumenwand des Blutgefäßes befindet;
eine biokompatible vernetzte Blutproteinzusammensetzung, wobei die biokompatible vernetzte Blutproteinzusammensetzung in vitro von Blut abgeleitet wird, welches dem Patienten, der unter Verwendung des Aneurysmabehandlungssystems behandelt wird, abgenommen wurde; und
ein Zubringungskatheter, welcher dazu geeignet ist, die biokompatible quervernetzte Blutproteinzusammensetzung in einen Raum zwischen der äußeren Wand des Stenttransplantats und dem Aneurysmasack einzubringen.

2. System nach Anspruch 1, wobei der Katheter ein Doppellumenkatheter ist.

3. System nach Anspruch 1, wobei der Katheter ein Tripellumenkatheter ist.

4. Aneurysmabehandlungssystem nach einem der vorangegangenen Ansprüche, wobei das biokompatible quervernetzte Blutprotein unter Verwendung von N-Hydroxyestern, Maleimiden, Haloacetylen, Pyridyldisulfiden, Kohlehydraten oder 1-Ethyl-3(3-Dimethylaminopropyl)-Carbodiimiden vernetzt wird.

5. Aneurysmabehandlungssystem nach einem der vorangegangenen Ansprüche, wobei die biokompatible quervernetzte Blutproteinzusammensetzung hauptsächlich aus Serumalbumin abgeleitet wird.

6. Aneurysmabehandlungssystem nach einem der Ansprüche 1 bis 3, wobei der Quervernetzer ein N-Hydroxysuccinimidester ist.

7. Aneurysmabehandlungssystem nach einem der vorangegangenen Ansprüche, wobei die Blutproteinzusammensetzung vor dem Quervernetzen durch das Erzeugen von Sulfhydrylgruppen an den Blutproteinen modifiziert wird.

8. Aneurysmabehandlungssystem nach einem der vorangegangenen Ansprüche, wobei die Blutproteinzusammensetzung vor dem Quervernetzen durch das Erzeugen von primären Amingruppen an den Blutproteinen modifiziert wird.

9. Aneurysmabehandlungssystem nach einem der vorangegangenen Ansprüche, wobei das Aneurysma, welches unter Verwendung des Aneurysmabehandlungssystems behandelt wird, ein Aortenaneurysma ist.

## Revendications

1. Système de traitement des anévrismes comprenant :
un stent à greffer pour mise en place dans un vaisseau sanguin en une position adjacente à un anévrisme ; le stent à greffer ayant une paroi intérieure et une paroi extérieure, la paroi extérieure étant adjacente à la paroi interne luminale du vaisseau sanguin après implantation dans le vaisseau sanguin en une position adjacente à l'anévrisme ;
une composition de protéines sanguines réticulées biocompatibles, la composition de protéines sanguines réticulées biocompatibles dérivant, in vitro, du sang prélevé depuis le patient soumis à un traitement par utilisation dudit système de traitement des anévrismes ; et
un cathéter de distribution convenant à la distribution de la composition de protéines sanguines réticulées biocompatibles dans un espace compris entre la paroi extérieure du stent à greffer et l'anévrisme.

2. Système selon la revendication 1, dans lequel le cathéter est un cathéter à double lumière.

3. Système selon la revendication 1, dans lequel le cathéter est un cathéter à triple lumière.

4. Système de traitement des anévrismes selon l'une quelconque des revendications précédentes, dans lequel la protéine sanguine réticulée biocompatible est réticulée en utilisant des N-hydroxyesters, des maléimides, des composés halogénoacétyle, des pyridyl-disulfures, des glucides ou le 1-éthyl-3-(3-diméthylaminopropryl)-carbodiimide.

5. Système de traitement des anévrismes selon l'une quelconque des revendications précédentes, dans lequel la composition de protéines sanguines réticulées biocompatibles dérive principalement de la sérum-albumine.

6. Système de traitement des anévrismes selon l'une quelconque des revendications 1 à 3, dans lequel l'agent de réticulation est un N-hydroxysuccinimide ester.

7. Système de traitement des anévrismes selon l'une quelconque des revendications précédentes, dans lequel la composition de protéines sanguines est modifiée, avant la réticulation, par génération de groupes sulfhydryle sur les protéines sanguines.

8. Système de traitement des anévrismes selon l'une quelconque des revendications précédentes, dans lequel la composition de protéines sanguines est modifiée, avant la réticulation, par génération de groupes amines primaires sur les protéines sanguines.

9. Système de traitement des anévrismes selon l'une quelconque des revendications précédentes, dans lequel l'anévrisme à traiter en utilisant le système de traitement des anévrismes est un anévrisme aortique.
